# EUROPEAN PATENT SPECIFICATION

(11) **EP 2 756 024 B1**
(45) Date of publication and mention of the grant of the patent: **18.11.2015**
(21) Application number: 12766070.2
(22) Date of filing: 25.09.2012
(51) Int. Cl.: C07C 69/708, C07C 59/135, C08G 65/22, C08G 65/00, C08G 65/26

(54) **LINEAR (PER)FLUOROPOLYETHERS WITH -CF(CF3)COF END GROUPS AND DERIVATIVES THEREOF**
LINEARE (PER)FLUORPOLYETHER MIT -CF (CF3)-COF-ENDGRUPPEN UND DERIVATE DAVON
POLYÉTHERS FLUORÉS OU PERFLUORÉS LINÉAIRES RENFERMANT DES GROUPES TERMINAUX-CF(CF3)COF ET LEURS DÉRIVÉS

(30) Priority: 03.10.2011 EP 11183716
(43) Date of publication of application: 23.07.2014
(73) Proprietor: Solvay Specialty Polymers Italy S.p.A., 20021 Bollate (MI) (IT)
(72) Inventor: FONTANA, Simonetta, Antonella, I-20142 Milano (IT); TONELLI, Claudio Adolfo Pietro, I-23877 Paderno D'adda (Lecco) (IT); GAVEZOTTI, Piero, I-20148 Milano (IT)
(74) Representative: Benvenuti, Federica
(86) International application number: PCT/EP2012/068884
(87) International publication number: WO 2013/050273

(56) References cited:
- JP-A- 2001 207 183
- US-A- 3 311 658

## Description

This application claims priority to European patent application No. 11183716.7 filed on October 3rd, 2011, the whole content of which is incorporated herein by reference for all purposes.

### Technical Field

The present invention relates to fluoropolyethers, in particular to linear (per)fluoropolyethers having functionalised end groups and to a process for preparing them.

### Background Art

Linear (per)fluoropolyethers containing -CF₂O-, -CF₂CF₂O-, -CF₂CF₂CF₂ O- and -CF₂CF₂CF₂CF₂O- units randomly distributed along the polymer chain are characterised by a lower glass transition temperature (Tg) than that of (per)fluoropolyethers containing also branched -CF(CF₃)- or -CF₂ CF(CF₃)- units, having pendant perfluoroalkyl groups. In particular, linear (per)fluoropolyethers containing, or essentially consisting of, -CF₂O- and -CF₂CF₂O- units are particularly flexible and are endowed with a Tg usually lower than -90°C, due to the presence of the -CF₂O- units.

Linear (per)fluoropolyethers containing -CF₂O- and -CF₂CF₂O- units are conveniently obtained by photopolymerization of tetrafluoroethylene in the presence of oxygen, followed by reduction of the peroxy groups present in the resulting polymer; this process leads to linear functionalised (per)fluoropolyethers containing -CF₂COF end groups, which can be converted into further functionalised (per)fluoropolyethers according to reactions known in the art. In these furher functionalised (per)fluoropolyethers, a functional group is linked to the (per)fluoropolyether chain through a -CF₂- moiety. Usually, these further functionalised (per)fluoropolyether are endowed with good chemical resistance; however, it has been observed that, under severe conditions, derivatives like esters or amides undergo hydrolysis.

US 4115367 (US AIR FORCE) 19.09.1978 and US 4064109 (US AIR FORCE) 20.12.1977 disclose thermooxidatively and hydrolytically stable perfluoroalkylether bisbenzoxazole polymers containing a perfluoroalkyl ether chain and benzoxazole end groups, wherein the benzoxazole end groups are linked to the perfluoroalkyl ether chain through -CF(CF₃)- units; however, the polymers disclosed in US 4115367 contain a perfluoroalkylether chain that does not comprise -CF₂O- units, while the polymers disclosed in US 4064109 contain a perfluoroalkylether chain containing -(CF)CF₃- units. Accordingly, these compounds have a Tg higher than that of linear (per)fluoropolyethers containing -CF₂O- units.

It is also noted that functionalised (per)fluoropolyethers in which functional end groups are linked to the (per)fluoropolyether chain through -CF(CF₃)-moieties can be prepared by addition of hexafluoropropylene oxide (HFPO) to (per)fluoropolyethers containing -COF end groups in the presence of suitable initiators; however, yields are poor, due to the fact that HFPO undergoes oligomerization, giving rise to mixtures of mono- or polyaddition products, i.e. products containing one or more HFPO or units at each end of the polymer chain these mixtures are difficult to separate. For instance, US 2004116742 (3M INNOVATIVE PROPERTIES CO) 17.06.2004 discloses a process for preparing linear fluorinated compounds, including (per)fluoropolyether compounds having a -CF(CF₃ )COF terminal group, by reaction of HFPO with a precursor having a -COF terminal group; however, also this process, which is carried out in the presence of a fluoride salt as catalyst and a polar solvent and which makes use of an excess of at least 10% precursor, affords poor yields, as it leads to a mixture that contains, in addition to a product containing one HFPO unit, products containing more HFPO units and a significant amount of unreacted precursor. In fact, US 2004116742 discloses only the separation of a monoaddition product obtained from a precursor of formula CF₃-O-CF₂CF₂COF and does not specifically disclose the preparation of (per)fluoropolyethers comprising a linear (per)fluoropolyether chain having two chain ends, each chain end comprising one HFPE end group.

US 4053498 (US AIR FORCE) 11.10.1977 , which relates to perfluoroalkylene ether-imidate and thioimidate esters, teaches to prepare diacyl fluorides of formula FOCF(CF₃)[OCF₂CF(CF₃)]ₘO(CF₂)₅[CF(CF₃ )CF₂O]ₙCF(CF₃)COF, wherein m + n is 4 or 5, by reaction of exafluoroglutaryl fluoride, in the presence of CsF as catalyst and tetraglyme as solvent, according to what disclosed in US 3250807 (DU PONT). According to this patent, acyl fluorides of formula:

FOC-R'_{f}-O[CF(CF₃)CF₂O]ₙCF(CF₃)COF

and

FOCCF(CF₃)[OCF₂CF(CF₃)]ₘOCF₂R'_{f}CF₂O[CF(CF₃)CF₂O]ₚCF(CF₃)CO F

wherein R'_{f} is a perlfuoroalkylene radical of 1 to 20 carbon atoms, n is a number from 0 to 35 inclusive and m and p are numbers whose sum is from 0 to 35 inclusive are prepared by reaction of mono- or diacyl fluorides with HFPO in a polar organic solvent, using a fluoride salt as catalyst in an amount which is at least 0.01% by weight of the HFPO, at temperatures ranging from -80°C to 200°C. This patent teaches that the degree of polymerization depends on various factors, namely the catalyst, the temperature and the acid fluoride/HFPO ratio; in particular, it teaches that a lower degree of polymerization is obtained at the low end of the temperature range and that the monoaddition product is obtained when the acid fluoride/HFPO ratio is one or greater than one. Nevertheless, it also states that the control on the amount of HFPO units inserted is not absolute and that products with higher or lower molecular weight can also be obtained.

US 3311658 (DU PONT DE NEMOURS AND CO.) discloses omega-iodofluorocarbon ether acid fluorides of formula:

I(CF₂)ₙ₊₁-O-[CF₂CF₂O]ₚ₊₁-[CF(CF₃)CF₂O]ₘ-CF(CF₃)C(O)F

wherein n is an integer from 1 to 8 inclusive, m is an integer from 1 to 5 inclusive and p is an integer from 1 to 5 inclusive. Such compounds are said to be capable of being converted into vinyl ethers which can in turn be copolymerized to provide fluorocarbon resins that can be cross-linked. The ethers alone are said to be useful as dispersing agents (col. 4, lines 34 - 37).

JP 2001207183 (MATSUSHITA ELECTRIC CO LTD) discloses a lubricant composition for magnetic recording media which comprises linear PFPE derivatives, among them PFPE carboxylic derivatives; however, such derivatives do not have carboxylic end groups bound to -CF(CF3)-moieties.

There is therefore the need to provide (per)fluoropolyethers that are endowed with both low Tg and high chemical resistance and that can be prepared according to a convenient process, which allows to control the number of HFPO units inserted at the polymer end(s).

### Summary of invention

The present invention relates to mono- or bi-functional (per)fluoropolyethers comprising a linear (per)fluoropolyether chain having two ends, wherein one or two ends contain -CF(CF₃)COF groups, to a process for preparing them and to their use as precursors in the preparation of further functionalised (per)fluoropolyethers. The invention also relates to these further functionalised (per)fluoropolyethers.

For the purposes of the present invention, the term "(per)fluoropolyether" means a (per)fluoropolymer containing a (per)fluoropolyether chain, i.e. a fully or partially fluorinated polyoxylakylene chain [herein after also referred to as (R_{f}) chain] which comprises, preferably consists of, recurring units having at least one catenary ether bond and at least one fluorocarbon moiety.

The (per)fluoropolyethers according to the present invention contain a linear (per)fluoropolyoxyalkylene chain (R_{f}) comprising, preferably consisting of, repeating units R°, randomly distributed along the (per)fluoropolyoxyalkylene chain, selected from the group consisting of:
(i) -CF₂O-;
(ii) -CF₂CF₂O-;
(iii) -CF₂CF₂CF₂O-;
(iv) -CF₂CF₂CF₂CF₂O-.

Preferably, the R_{f} chain contains, preferably consists of, both -CF₂O- and -CF₂CF₂O- units.

For the avoidance of doubt, in the present description, the term (per)fluoropolyether indicates a fully or partially fluorinated polyether and the acronym "PFPE" stands for (per)fluoropolyether as defined hereinabove. Typically, the mono- or bifunctional (per)fluoropolyethers having -CF(CF₃ )COF end groups according to the present invention comply with formula (I) below:

X-O-R_{f}-CF₂CF₂O-[CF(CF₃)CF₂O]_{X}-CF(CF₃)-COF (I)

wherein:
- R_{f} is as defined above and
- X is -CF₂CF₂O-[CF(CF₃)CF₂O]ₓ-CF(CF₃)-COF or a (per)fluoroalkyl chain containing from 1 to 3 carbon atoms, optionally containing 1 chlorine, bromine or hydrogen atom; and
- x is 0 or an integer equal to or higher than 1.

Preferably, chain R_{f} complies with formula: (CF₂O)ₙ(CF₂CF₂O)ₘ, in which m and n are 0 or integers equal to or higher than 1, preferably ranging from 1 to 4 and preferably selected in such a way that the number average molecular weight ranges from 400 to 10,000, preferably from 600 to 5,000, with the proviso that at least one of m and n is other than 0; preferably, m and n are other than 0 and the m/n ratio ranges from 0.1 to 10, preferably from 0.2 to 5, more preferably from 0.1 to 2.5.

In a first preferred embodiment, x is 0. In a second preferred embodiment, x is an integer equal to or higher than 1; for example, x may range from 1 to 100, preferably, from 1 to 50, more preferably from 1 to 20, even more preferably from 1 to 5.

The compounds of formula (I) in which x is 0 can be prepared by reaction of a compound (herein after also referred to as acyl precursor) of formula (II):

X¹-O-R_{f}-CF₂COF (II)

in which:
- R_{f} is as defined above and
- X¹ is CF₂COF or a (per)fluoroalkyl chain containing from 1 to 3 carbon atoms, optionally containing 1 chlorine, bromine or a hydrogen atom with HFPO in the presence of an inorganic or organic fluoride as catalyst and of a mixture of a fluorinated solvent and an oxygen-containing hydrogenated solvent.

It has indeed been observed that, thanks to the use of such a solvent mixture, the process according to the invention has a high mono-addition selectivity, usually higher than 90%, i.e. it allows to insert only one HFPO unit at each -COF end group of the acyl fluoride precursor of formula (II) as defined above. Furthermore, the process allows to achieve more than 90% conversion of the -COF groups of the acyl precursor of formula (II) into -CF(CF₃)COF groups.

The acyl fluoride precursor of formula (II) above can be synthesised according to US 3847978 A (MONTEDISON SPA), US 5164517 (AUSIMONT SPA) or US 5371272 (AUSIMONT SPA)

The inorganic fluoride catalyst used in the preparation of the compounds of formula (I) as defined above is typically selected from LiF, NaF, KF, CaF ₂, BaF2, MgF₂, CsF₂; according to a preferred embodiment, the inorganic fluoride is CsF. The organic fluoride catalyst can be, for instance, a quaternary alkylammonium fluoride or an alkali metal perfluoroalkoxide. A preferred example of alkylammonium fluoride is tetrabutylammonium fluoride. The molar amount of catalyst typically ranges from 0.1 to 100%, preferably from 0.1 to 50%, more preferably from 0.5 to 30% with respect to the equivalents of acyl fluoride precursor (II).

Typically, the equivalent ratio between the acyl fluoride precursor (II) and HFPO ranges from 1:1.1 to 1:3, preferably from 1:1.3 to 1:1.8, while the weight ratio of fluorinated solvent to oxygen-containing hydrogenated solvent typically ranges from 0.1 to 10, preferably from 0.5 to 2. The reaction is usually carried out at a temperature ranging from -40°C to 100°C, preferably from -30 to +40°C, more preferably from -25°C to +20°C; pressure usually ranges from ambient pressure to 1013.25 kPa.

Upon completion of the reaction, the solvents, any unreacted HFPO, any by-products are removed by distillation, either under ambient pressure or at reduced pressure, according to the boiling point of the solvents used.

The fluorinated solvents to be used in the process according to the present invention are usually selected from hexafluoroxylene (bis-trifluoromethylbenzene), hydrofluoroethers, for example those marketed as Novec^{®} by 3M^{®} and hydrofluoropolyethers marketed as H-Galden^{®} by Solvay Solexis. As oxygen-containing hydrogenated solvents, (poly)alkylene glycol ethers like diethylene glycol dimethyl ether and tetraethylene glycol dimethyl ether can be mentioned in particular.

The compounds of formula (I) in which x is equal to or higher than 1 can be prepared by submitting a compound of formula (I) in which x is 0 to reaction with one or more equivalents of HFPO under the conditions described above. These compounds are particularly useful in the preparation of further functional derivatives used for imparting hydro- or oleo-repellency, because the presence of a plurality of HFPO end units lowers surface energy and improves barrier effect.

The compounds of formula (I) can be used as precursors of functionalised (per)fluoropolyethers in which functional groups other than -COF are linked to one or to both ends (depending on whether the compound of formula (I) is mono- or bifunctional) of the (per)fluoropolyether chain through one or more HFPO units and, optionally, through a linking bridge. These functional groups are such as to confer reactivity towards co-reactants or substrates, so that further compounds can be obtained or so that substrate surfaces can be modified. Substrates can be both natural and synthetic; among them, paper, cotton, wood, stony materials, polymeric materials, metal or inorganic substrates can be mentioned.

Typically, the functionalised (per)fluoropolyethers according to the present invention which can be obtained from compounds (I) comply with formula (III) below:

X²-O-R_{f}-CF₂CF₂O-[CF(CF₃)CF₂O]ₓ-CF(CF₃)-(A)_{q}-(T)ₚ (III)

in which:
- X² is -CF₂CF₂O-[CF(CF₃)CF₂O]ₓ-CF(CF₃)-(A)_{q}-(T)ₚ or a (per)fluoroalkyl chain containing from 1 to 3 carbon atoms, optionally containing 1 chlorine, bromine or hydrogen atom;
- R_{f} and x are as defined above;
- q and p are 0 or 1, with the proviso that at least one of them is other than 0;
- A is a straight or branched alkyl chain, an (alkyl)clycloaliphatic or an (alkyl)aromatic chain when p is 0 or a straight or branched alkylene chain, an (alkylene)clycloaliphatic or an (alkylene)aromatic chain when p is 1;
- T is a group consisting of or containing one or more groups selected from hydroxyl, ester, carboxy, thiol, alkylthio, amino, alkylamino, a silicon-containing group, cyano, isocyanate, alkenyl, vinyl, vinyl ether, vinylalkyl ether, aldehyde, keto, sulphonyl, sulphate, phosphonate, phosphate, carbonate, anhydride, halogen, oxime, hydrazine, guanidine, amide, acrylate, methacrylate, nitro and epoxide.

With regard to the definition of A:
- a straight or branched alkyl or alkylene chain according to the present invention is preferably a straight or branched alkyl or alkylene chain containing from 1 to 20 carbon atoms; an (alkyl)cycloaliphatic or (alkylene)cycloaliphatic chain in which the alkyl or alkylene moiety is a straight or branched alkyl or alkylene moiety containing from 1 to 20 carbon atoms and the cycloaliphatic moiety has from 3 to 20 carbon atoms; an (alkyl)aromatic or (alkyene)aromatic chain in which the alkyl or alkylene moiety is as defined above and the aromatic moiety contains 5 to 20 carbon atoms. The alkyl or alkylene chain optionally contains one or more -COO-, -COS- or -CONH- groups and/or one or more etheroatoms selected from N, P, S and O and in the cycloaliphatic or aromatic moiety one ore more carbon atoms is optionally replaced by etheroatoms selected from N, P, S and O.

Preferred groups A in which the alkyl or alkylene chain contains one or more O atoms are (poly)alkyleneoxide chains containing repeating units of formula -CH₂CH₂O-, -CH₂CH(CH₃)O-, -(CH₂)₃O- or -(CH₂)₄O-, more preferably repeating units of formula -CH₂CH₂O-, -CH₂CH(CH₃)O-.

With regard to the definition of T:
- a group containing more hydroxy groups is preferably a group of formula -CH(CH₂OH)₂;
- an ester group is preferably a group of formula -COOR' in which R' is a straight or branched alkyl chain containing from 1 to 20 carbon atoms and optionally containing fluorine atoms and/or a 3 to 20 member cycloaliphatic or heterocycloaliphatic moiety or a 5 to 20 member aromatic or heteroaromatic moiety;
- a group containing more ester groups is preferably a group of formula -CH(COOR')₂, in which R' is as defined above;
- the term "carboxy" comprises also its alkali and alkali metal salts and acyl halides, preferably acyl chlorides;
- a group containing more carboxy groups is preferably a group of formula -CH(COOH)₂;
- an alkylthio group is preferably a group of formula -SR', in which R' is as defined above;
- a silicon-containing group is preferably a group of formula -SiR'*a*Q_{3-d} in which R' is as defined above, *d* is 0 or an integer from 1 to 3, Q is an -O(CO)*d0*R' group and *d0* is 0 or 1;
- a group containing more cyano groups is preferably a group or formula -CH(CN)₂;
- a vinyl alkyl ether group is preferably a group complying with formula -O(CH₂)_{m'}CH=CH₂, in which m' ranges from 1 to 18; more preferably, a vinyl ether group is a group complying with formula -OCH₂CH=CH₂. A group containing more vinyl ether groups is preferably a group of formula -CH[O(CH₂)_{m'}CH=CH₂]₂, in which m' is as defined above, more preferably a group of formula -CH[OCH₂CH=CH₂]₂;
- an alkylamino group comprises a monoalkylamino group preferably complying with formula -NHR' and a dialkylamino group preferably complying with formula -NR'₂ in which R' is as defined above; a group containing more amino groups is preferably a group of formula -CH(CH₂NHR')₂ or a group of formula -CH(CH₂NR'₂)₂ in which R' is as defined above;
- the term aldehyde is meant to comprise also the corresponding acetal and thioacetal derivatives, such as dimethyl acetals and dimethylthioacetals;
- a keto group is preferably a group of formula -COR' in which R' is as defined above, including any ketal and thioketal derivatives, such as dimethyl acetals and dimethylthioacetals;
- a sulphonyl group is preferably a group of formula -SO₃H and any alkali or alkaline-hearth metal salts; -SO₂Hal, in which Hal is halogen as defined above, preferably chlorine; a group of formula -SO₂R', in which R' is as defined above; a group of formula -SO₂R", in which R" is a fluorinated straight or branched C₁-C₆alkyl chain, more preferably a group of formula -SO₂CF₃;
- a sulphate group is preferably a group of formula -OSO₃H and any alkali or alkaline-hearth metal salts, -OSO₂Hal or -OSO₂R', in which Hal and R' are as defined above;
- a phosphonate group is preferably selected from -P(O)(OH)₂ and -P(O)(OH)(OR') and their respective alkali or alkaline-hearth metal salts; and -P(O)(OR')₂, in which R' is as defined above;
- a phosphate group is preferably selected from -OP(O)(OH)_{2;} -OP(O)(OH)(OR') and their respective alkali or alkali-earth metal salts and -OP(O)(OR')₂; in which R' is as defined above.
- a carbonate group is preferably selected from -OC(O)OH and its alkali or alkali-earth metal salts, -OC(O)Hal in which Hal is as defined above and
- OC(O)OR', in which R' is as defined above;
- halogen is selected from fluorine, chlorine, bromine and iodine, iodine being particularly preferred;
- an anhydride group is preferably a group of formula -(O)C-O-C(O)-R', in which R' is as defined above;
- an oxime group is preferably a group of formula -NH-OH or -NH-OR', in which R' is as defined above;
- a hydrazino group is preferably a group of formula -NH-NH₂ or a group or formula -NH-NHR', in which R' is as defined above;
- a guanidine group is preferably a group of formula -NH-C(=NH)-NH₂ or a group of formula -NH-C(=NH)-NHR', in which R' is as defined above.

A first particularly preferred group of compounds of formula (III) above is represented by compounds in which q is 0, p is 1 and T is a -COOR' group in which R' is defined above. These compounds, herein after referred to as compounds or esters (III) of formula:

X²-O-R_{f}-CF₂CF₂O-[CF(CF₃)CF₂O]ₓ-CF(CF₃)-COOR' (IIIa)

in which X², R_{f} x and R' are as defined above
can be conveniently obtained from a compound of formula (I) as defined above by reaction with an alcohol R'OH, in which R' is as defined above, according to known methods. Most conveniently, after the synthesis of a compound of formula (I) according to the procedure reported above, the solvents, any unreacted HFPO and any by products are not removed from the reaction mixture and an alcohol R'OH is added; in a preferred embodiment, the alcohol is ethanol. A tertiary amine can also be added as an HF scavenging agent.

Esters (IIIa) can be conveniently used as precursors of other compounds of formula (IIIa) or of further derivatives.

Esters (IIIa) can be, for instance, hydrolysed according to known methods to provide the corresponding carboxylic acids, i.e. compounds of formula (III) in which q is 0, p is 1 and T is -COOH. These compounds will be also herein after referred to as compounds or acids (IIIb), having formula:

X²-O-R_{f}-CF₂CF₂O-[CF(CF₃)CF₂O]ₓ-CF(CF₃)-COOH (IIIb)

in which X², R_{f} and x are as defined above.

Acids (IIIb) can be used as such or in the form of derivatives like salts, acyl halides, anhydrides of further reactive compounds, for the preparation of further compounds of formula (III) or derivatives thereof.

The preparation of exemplary functional derivatives of formula (III), in particular amido-containing derivatives, starting from esters (IIIa) and acids or acid derivatives (IIIb) is schematically resumed in table 1 below. This tables and the following tables 2-4 report the co-reagent and the -(A)p-(T)q group in the resulting compound (III).

**Table 1**

| | **Co-reagent** | **Group -(A)p-(T)q- in the resulting compound (III)** |
|---|---|---|
| 1 | H₂NCH₂-CH=CH₂ | -CONHCH₂-CH=CH₂ |
| 2 | H₂N(CH₂)₃CO₂H | -CONH(CH₂)₃CO₂H |
| 3 | H₂N(CH₃)CH₂CH₂OH | -CON(CH₃)CH₂CH₂OH |
| 4 | H₂NCH₂CH₂NH₂ | -CONCH₂CH₂NH₂ |
| 5 | H₂NCH₂CH₂SH | -COHNCH₂CH₂SH |
| 6 | 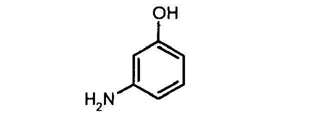 | 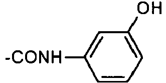 |
| 7 | 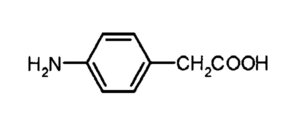 | 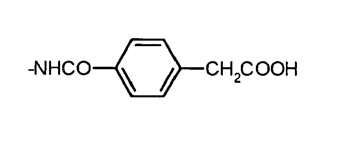 |
| 8 | 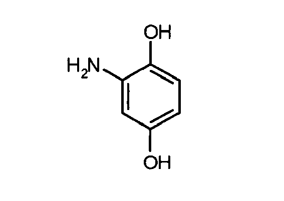 | 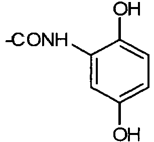 |
| 9 | 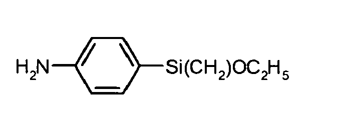 | 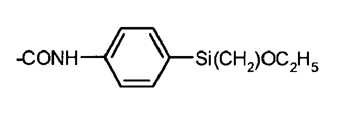 |
| 10 | 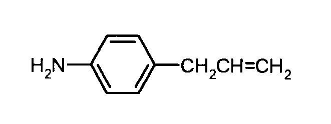 | 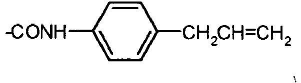 |
| 11 | 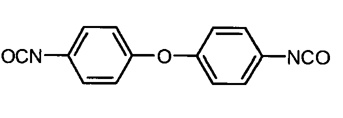 | 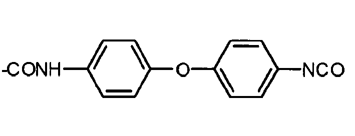 |
| 12 | 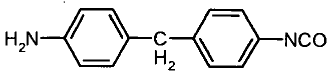 | 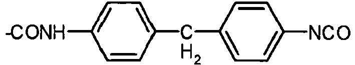 |
| 13 | 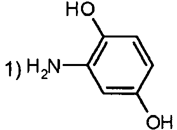 | 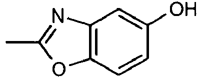 |
| | 2) heating | |
| 14 | 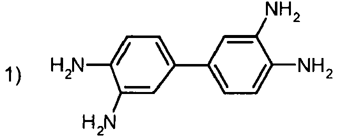 | 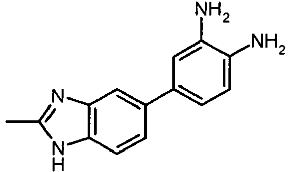 |
| | 2) heating | |
| 15 | 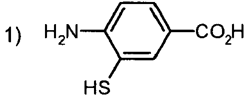 | 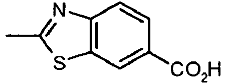 |
| | 2) heating | |
| 16 | H₂NHCOC(CH₃)=CH₂ | 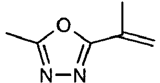 |
| 17 | HOCH₂C(CH₃)₂CH₂OH | -CO₂CH₂C(CH₃)₂CH₂OH |
| 18 | 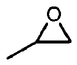 | -CO₂CH₂CH(OH)CH₃ |
| 19 | CH₂=CHCH₂OH | -CO₂CH₂CH=CH₂ |
| | 1) NH₃ | -CN |
| | 2) dehydration | |
| 20 | 1) NH₃ | 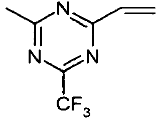 |
| | 2) dehydration | |
| | 3) | |
| | 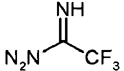 | |
| | 4) (CH₂=CHCO)₂O | |

Esters (IIIa) can also be reduced according to known methods to provide compounds of formula (III) in which q is 0, p is 1 and T is a -CHO group, hereinafter also referred to as compounds or aldehydes (IIIc), having formula:

X²-O-R_{f}-CF₂CF₂O-[CF(CF₃)CF₂O]ₓ-CF(CF₃)-CHO (IIIc)

in which X², R_{f} x and R' are as defined above.
Aldehydes (IIIc) can in turn be used as such or in the form of their respective acetals or thioacetals as precursors of other compounds of formula (III) or further derivatives.

The preparation of functional derivatives of formula (III) from aldehydes (IIIc) is schematically illustrated in table 2 below:

**Table 2**

| | **Co-reagent** | **Group -(A)p-(T)q- in the resulting compound (III)** |
|---|---|---|
| 1 | CH₂=P(C₆H5)₃N | -CH=CH₂ |
| 2 | CH₃OH, acid | -CH(OCH₃)(OH) |
| 3 | NH₃ | -CH(NH₂)(OH) |
| 4 | NH₃, -H₂O | -CH=NH |
| 5 | 2NH₃ | -CH(NH₂)NH₂ |

Esters (IIIa) can also be reduced according to known methods to provide compounds of formula (III) in which A is -CH₂-q is 1, p is 1 and T is a -OH group, herein after also referred to as compounds or alcohols (IIId), having formula:

X²-O-R_{f}-CF₂CF₂O-[CF(CF₃)CF₂O]ₓ-CF(CF₃)-CH₂OH (IIId)

in which X², R_{f} and x are as defined above.

This reduction can be carried out according to conventional methods and with conventional reagents, for example by reaction with a hydride, preferably NaBH₄ or LiAlH₄, more preferably NaBH₄.

Alcohols (IIId) can be in their turn used as precursors of other compounds of formula (III) or of further derivatives, according to conventional methods and with conventional reagents; for example, they can be transformed into compounds in which q is 0, p is 1 and T is thio, amino or carbonate. The -OH group can also be transformed into a leaving group, typically a (per)fluoroalkylsulfonyl group like CF₃SO₂O⁻, as illustrated in table 3, entry 5 below, or CH₃(CF₂)₃-SO₂O-, and the resulting compound can be reacted with a nucleophile compound, as illustrated in table 4 below. Alcohols (IIId) can also be used as nucleophile reagents with compounds bearing a leaving group or they can be reacted with organic acids, such as carboxylic acids, to provide ester derivatives. Alcohols (IIId) can also be reacted, for example, with one or more ethylene or propylene oxide units in order to provide compounds of formula (III) in which p and q are 1, A is a straight or branched aliphatic chain containing one or more oxygen atoms and T is -OH; these hydroxyalkyleneoxide compounds can be represented by formula (IIIe)

X²-O-R_{f}-CF₂CF₂O-[CF(CF₃)CF₂O]ₓ-CF(CF₃)-CH₂O-(CHYCHYO)_{y}H (IIIe)

in which X², R_{f} and x are as defined above and Y is hydrogen or methyl and y is an integer equal to or higher than 1, preferably an integer ranging from 1 to 5, more preferably 1.

Compounds (IIIe) can in turn be useful for other functional derivatives of formula (III) in which T has one of the meanings defined above other than oxygen or for the preparation of further functional compounds, according to reactions known in the art, for example they can be subjected to the same reactions as those mentioned above for alcohols (IIId).

The preparation of exemplary functional derivatives of formula (III) starting from alcohols (IIId) is schematically represented in table 3 below.

**Table 3**

| | **Co-reagent** | **Group -(A)p-(T)q- in the resulting compound (III)** |
|---|---|---|
| 1 | 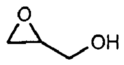 | -CH₂OCH₂CH(OH)CH₂OH |
| 2 | epibromidrine | 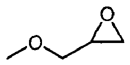 |
| 3 | CH₂=CHCH₂Br | CH₂=CHCH- |
| 4 | 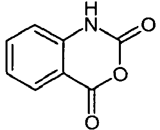 | 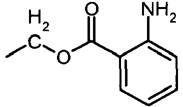 |
| 5 | CF₃SO₂F + (C₂H₅)₃N | -CH₂OSO₂CF₃ |
| 6 | NCCl + (C₂H₅)₃N | -CH₂OCN |
| 7 | CH₂=C(CH₃)C(O)Cl | -CH₂OC(O)C(CH₃)=CH₂ |
| 8 | NaN₃ | -NCO |

Table 4 below schematically represents the preparation of exemplary compounds of formula (III) that can be prepared starting from an alcohol (IIId) wherein the -OH group has been converted into a leaving group, such as a -CH₂OSO₂CF₃ group inserted according to entry 5 of table 3.

**Table 4**

| | **Co-reagent** | **Group -(A)p-(T)q- in the resulting compound (III)** |
|---|---|---|
| 1 | 1) | 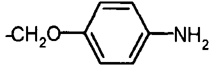 |
| | 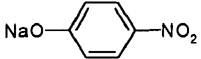 | |
| | 2) H₂ | |
| 2 | 1) | 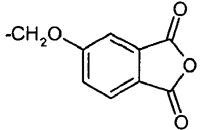 |
| | 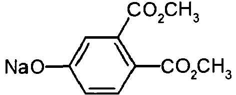 | |
| | 2) hydrolysis | |
| | 3) acetic anhydride | |
| 3 | 1) | 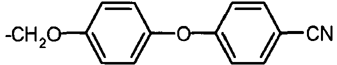 |
| | 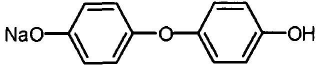 | |
| | 2) BrCN triethylamine | |
| 4 | 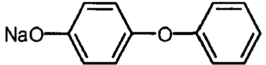 | 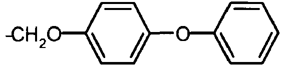 |
| 5 | NH3 | -CH₂NH₂ |
| 6 | CH₃NH₂ | -CH₂NHCH₃ |
| 7 | Nal | -CH₂l |
| 8 | 1) CH₃COSNa | -CH₂SH |
| | 2) hydrolysis | |

Table 5 below schematically represents the conversion of certain functional derivatives obtained according to table 4 above into further functional derivatives.

**Table 5**

| **Functional group -A-T- in starting compound (III)** | **Co-reagent(s)** | **Functional group -A-T- in final compound (III)** |
|---|---|---|
| 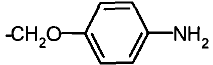 | Phosgene | 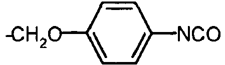 |
| -CH₂NH₂ | Phosgene | -CH₂NCO |
| -CH₂NH₂ | 1) HCO₂CH₃ | -CH₂N⁺≡C- |
| | 2) COCl₂ + TEA | |
| -CH₂NH₂ | 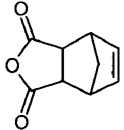 | 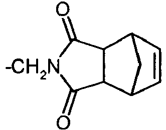 |
| 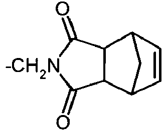 | HSi(CH₃)₂OCOCH₃ + H₂PtCl₆ | 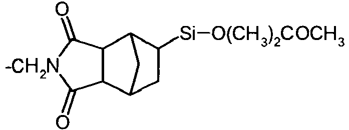 |
| -CH₂NHCH₃ | BrCN, TEA | -CH₂N(CN)CH₃ |
| -CN | CH₃OH, TEA | -C(OCH₃)=NH |
| -CN | NH₃ | -C(NH₂)=NH |
| -CN | HN₃ | 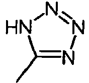 |
| -CH₂SH | Cl₂, H₂O | -CH₂SO₂Cl |
| 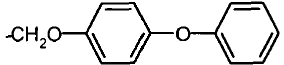 | CISO₃H | 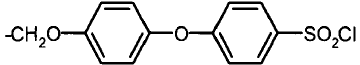 |

Additional preferred examples of further functional derivatives that can be prepared from the compounds of formula (III) are polyesters, polyamides, polyurethanes, polyacrylates and phosphazenes.

Polyesters can be prepared according to known methods from the compounds of formula (III) in which T is -OH or a hydroxyl-containing group as defined above by reaction with a polycarboxylic acid, preferably a dicarboxylic acid, according to methods known in the art. Polyesters can also be prepared from compounds of formula (III) in which T is a carboxy or a carboxy-containing group as defined above or an ester or ester-containing group as defined above with a polyalcohol, typically a diol, according to methods known in the art.

Polyamides can be prepared according to known methods by reaction of compounds of formula (III) in which T is a carboxy or a carboxy-containing group as defined above or an ester or ester-containing group as defined above with a polyamine, typically a diamine, according to methods known in the art. In a preferred embodiment, the diamine is selected from hexamethylenediamine, diethylenediamine and ethylenediamine.

Polyacrylates can be prepared according to known methods from the compounds of formula (III) in which T is acrylate or (meth)acrylate by radical polymerization with an acrylic or (meth)acrylic acid derivative in the presence or a radical initiator, according to methods known in the art.

Polyurethanes can be prepared according to known methods by reaction of compounds of formula (III) in which T is a group of formula -OH or a hydroxyl-containing group with a diisocyanate or a polyisocyanate, optionally in the presence of a chain extender selected from a diol or a diamine or a mixture thereof, according to methods known in the art.

Phosphazene derivatives can be prepared, for example, by reactions of compounds of formula (III) in which T is a group of formula -OH or a hydroxyl-containing group with a cyclic phosphazene of formula (IV) or (V) below: 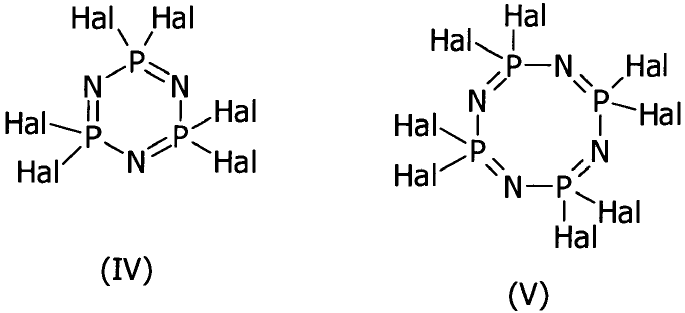 according to known methods, such as those disclosed in EP 1336614 A (SOLVAY SOLEXIS SPA) or in EP 0287892 A (HITACHI METALS LTD [JP]; MARUWA BUSSAN KK [JP), followed by hydrolysis by treatment with a base in an alcoholic medium.

The compounds of formula (III) or derivatives thereof can be used in a variety of applications; one of them is the treating of surfaces when it it desidered to impart hydro- or oleo-repellency to synthetic or natural substrated. Accordingly, the present invention further related to manufactured or synthetic articles treated with a compound of formula (III).

The invention will be now illustrated in greater detail in the following experimental section and non-limiting examples.

Should the disclosure of any patents, patent applications, and publications which are incorporated herein by reference conflict with the description of the present application to the extent that it may render a term unclear, the present description shall take precedence.

### EXPERIMENTAL SECTION

### 1. Materials and methods

The acyl precursors of formula (II) were synthesised according to US 3,847,978; other chemical and solvents were commercially available; in some instances, the solvents were distilled before use.

### 1.1 Determination of molecular weight and structure

Molecular weight and structure were determined by ¹H-NMR and ¹⁹ F-NMR analysis. The spectra were recorded on solvent-free samples, by means of a Varian Mercury instrument operating at 288 and 300 MHz with respect to the product. Chemical shifts are reported with respect to CFCl-₃ and TMS used as external standards.

### 1.2. Determination of the glass transition (Tg)

Glass transition was determined according to ASTM D3418, the standard method for determining the transition temperature of polymers through thermal analysis (DSC). DSC analysis was carried out using a Perkin-Elmer Pyris 2 instrument under He atmosphere.

### 1.3. Titration

Titration was carried out using Potentiometric titrator Tritini 716 GPD (Metrohm) and a Glass Electrode Metrohm 6.0262.100 (pH 0-13/0-80°C -3M KCl). The purpose of the method consisted in the determination of the percentage of the terminal groups (ester or amido groups) hydrolized under neutral or basic conditions. Details of the procedure are reported in the following paragraph.

### 1.4. Stability to hydrolysis

A selected amount of compound of formula (III) or a polymeric material obtained therefrom was immersed in water at constant pH, at temperature usually ranging from room temperature to 75°C and for a time usually ranging from 6 to 8 hours, typically of 7 hours; thereafter, the tested compound of formula (III) or material was submitted to ¹⁹-NMR quantitative analysis and to titration of the hydrolysed groups. The procedures reported below were followed for evaluating the stability of esters or amides complying or obtained from the compounds of formula (III) (test compounds). In such procedures, hydrolysis is expressed as equivalent percentage of hydrolysed groups.

### Stability test at neutral pH

A round-bottom two-neck flask, equipped with magnetic stirrer, condenser and thermometer, was charged with 2 g test compound and 10 ml distilled water. This mixture was then heated at 70°C and left under stirring at this temperature for 7 hours.

Thereafter, the mixture was cooled down to room temperature and the hydrolysis percentage was determined measuring the amount of resulting (in the case of esters compounds) or the amount of ammonium salt (in the case of amido compounds) by direct titrimetric analysis, using a 0.02 N solution of triethylamine in methanol or a 0.1 N solution of tetrabutylammonium hydroxide in isopropyl alcohol respectively.

### Stability test at basic pH

1 g test compound and 1.5 equivalents NaOH (0.1 M solution) were charged in a flask equipped with magnetic stirrer and condenser. The resulting mixture was left under stirring for 7 hours, unless indicated otherwise. In the case of esters, the hydrolysis percentage was determined through titration of the residual NaOH with a 0.1 N HCl solution in isopropanol, while in the case of amides it was determined by treatment with an excess of HCl and back titration of the resulting ammonium salt with a 0.1 N solution of tetrabutylammonium hydroxide in isopropyl alcohol.

### 2. Definitions

In the following examples, the expression "title compound" refers to the compound indicated in the title of each example; the expression "the compound of example (example number) refers to the compound indicated in the title of each example. The expressions "mono addition product" or "mono adduct" indicate the product obtained by addition of one HFPO unit at one or two ends of the mono or di-acylfluoride of formula (II) used as precursor in each example. The expression "bis-addiction product "and "bis adduct" indicate the product obtained by addition of two HFPO units at one or both ends of the mono or di-acylfluoride precursor. Likewise, the expressions "mono addition" or "mono addition reaction" indicate a reaction whereby one HFPO unit is added at one or two ends of the mono-or di- acylfluoride of formula (II) respectively used as precursor in each example and the expression "poly addition" or "polyaddition reaction" indicate a reaction whereby more than one HFPO unit is added at one or two polymer ends of the mono- or di- acylfluoride precursor.

### EXAMPLESaccording to the invention

### Example 1 - Synthesis of: FC(O)CF(CF₃)OCF₂CF₂O(CF₂CF₂O) m(CF20)n CF₂CF₂OCF(CF₃)COF (MW 1960)

### Method 1

1.85 g CsF (MW= 52, 12.2 mol), 70 g diethylene glycol dimethyl ether, 140 g bis trifluoromethyl benzene and 50.5 g diacyl fluoride of formula FC(O)CF₂O(CF₂CF₂O)ₘ(CF₂O)ₙCF₂COF (M_W= 1650, Mw/Mn = 1.2, m/n=1.5, 30.6 mmol) were charged under nitrogen atmosphere into an 0.5 I AISI 316 reactor equipped with agitator, sampling device, thermocouple and manometer. The resulting mixture was heated to 40°C and left under stirring for two hours, then cooled down to -25°C and nitrogen was removed by connecting the reactor to a vacuum pump. 15 g hexafluoropropylene oxide (MW=166, 90.4 mmol) was added over 2 hours and the resulting mixture was left under stirring for 4 hours.

After this time, the mixture was allowed to warm up to room temperature and 3.5 g of a low-boiling product were recovered in a dry ice trap; ¹⁹F NMR confirmed that the product was CF₃CF₂COF. The reaction crude was then poured into a separatory funnel under inert atmosphere; separation of two phases, an upper inorganic phase and a lower organic phase was observed. The latter was recovered and distilled, to provide the following fractions:
- 0.5 g CF₃CF₂CF₂OCF(CF₃)COF (b.p. 55°C);
- 130 g bis trifluoromethyl benzene (b.p. 112°C);
- 10 g diethylene glycol dimethyl ether (b.p. 160°C) and
- a distillation residue.

The distillation residue was submitted to thin layer distillation at a temperature ranging from 200 to 250°C at a pressure lower than 1.33 Pa, to provide 56.3 g FC(O)CF(CF₃)OCF₂CF₂O(CF₂CF₂O)ₘ(CF₂O)ₙCF₂CF₂ OCF(CF₃)COF (MW 1960, 28 mmol) containing:
- 2% FC(O)CF₂O(CF₂CF₂O)ₘ(CF₂O)ₙCF₂CF₂O-CF(CF₃)COF (MW 1800) and
- 2% of a product having one HFPO unit at one chain end and two HFPE units [i.e. a moiety of formula -CF(CF₃)CF₂OCF(CF₃)COF] at the other chain end and
- a thin layer distillation residue.

The thin layer distillation residue (4.8 g) was analysed by ¹⁹F-NMR; the results confirmed that the residue consisted of:
- FC(O)CF(CF₃)OCF₂CF₂O(CF₂CF₂O)ₘ(CF₂O)ₙCF₂CF₂O-CF(CF₃) CF₂OCF(CF₃)COF (MW 2120) (81%) in admixture with
- FC(O)CF(CF₃)OCF₂CF₂O(CF₂CF₂O)ₘ(CF₂O)ₙCF₂CF₂O-CF(CF₃)COF (MW 1960) (19%).

The yield of title compound with respect to the starting diacyl fluoride was 91.4%; purity was 96% and Tg (midpoint) was -110°C.

The overall mono-addition selectivity (expressed as % of meq of -CF₂COF end groups reacted with one HFPO unit with respect to the meq of -CF₂ COF end groups reacted with more HFPO units) was 96% .

### Method 2

The synthesis was carried out according to method 1, using 1.78 g CsF (MW = 152, 11.7 mmol), 70 g diethylene glycol dimethyl ether, 120 g o H Galden^{®} PFPE (grade A), 50 g diacyl fluoride of formula FC(O)CF₂O (CF₂ CF₂O)ₘ(CF₂O)ₙCF₂COF (MW= 1650, Mw/Mn = 1.2, m/n=1.5, 30.3 mmol) and 15.5 g HFPO (MW=166, 93.4 mmol).

Upon completion of the reaction, the low-boiling fluorinated by-products and the solvents were removed by distillation at room pressure as described in method 1. The distillation residue was submitted to thin layer distillation, to provide 55.2 g of a mixture containing: 95% title compound (MW 1960, 28.3 mmol) in admixture with:
- 3% of a compound having one HFPO unit at one chain end and a moiety of formula -CF(CF₃)CF₂0CF(CF₃)COF at the other chain end and
- 2% of a compound resulting from the reaction of only one -COF group of the diacyl fluoride precursor.

The thin layer distillation residue (approx. 5.2 g) was analysed by ¹⁹ F-NMR; the results confirmed that this residue consisted of:
- 77% of a compound containing one HFPO unit at one chain end and a moiety of formula -CF(CF₃)CF₂OCF(CF₃)COF at the other chain end;
- 2% of a compound resulting from the reaction of only one -COF group of the diacyl fluoride precursor;
- 23% title compound.

The yield of title compound amounted to 88% and the selectivity of mono-addition (defined as in method 1) was 96%. Conversion was therefore higher than 90%.

### Method 3

Method 2 was followed, using 0.3 g CsF (MW = 152, 2 mmol), 70 g diethylene glycol dimethyl ether, 140 g bis-trifluoromethyl benzene, 55 g diacyl fluoride of formula FC(O)CF₂O(CF₂CF₂O)ₘ(CF₂O)ₙCF₂COF (MW = 1650, Mw/Mn = 1.1, m/n=1.5, 33.3 mmol) and 15 g HFPO (MW=166, 90 mmol), with the difference that, before addition of HFPO, temperature was lowered to 20°C and that, after the addition, the mixture was stirred at this temperature for 8 hours.

Upon completion of the reaction, the low-boiling fluorinated by-products and the solvents were removed by distillation at room pressure as described in method 1, to provide 62.1 g of a product containing:
- 97% title compound;
- 2% of a compound having one HFPO unit at one chain end and a moiety of formula -CF(CF₃)CF₂OCF(CF₃)COF at the other chian end and
- 1% of a compound of formula: FC(O)CF₂O(CF₂CF₂O)ₘ(CF₂O)ₙCF₂CF₂ O-CF(CF₃)COF (MW = 1800),
as confirmed by ¹⁹F-NMR analysis.

The distillation residue (approx. 4.9 g) was analysed by ¹⁹F-NMR and the results confirmed that it contained 15% title compound.

The overall yield of title compound was 92.3% (97% purity) and the selectivity of mono-addition (as defined in method 1) was 96%. Conversion was therefore higher than 90%.

### Example 2 - Synthesis of FC(O)CF(CF₃)OCF₂CF₂O(CF₂CF₂O)-ₘ(CF₂O)ₙCF₂CF₂OCF(CF₃)COF (MW 1080)

This compound was synthesised according to method 2, using 2.0 g CsF (MW=152, 13 mmol), 70 g diethylene glycol dimethyl ether, 110 g of H Galden^{®} PFPE (grade A), 50 g diacyl fluoride of formula FC(O)CF₂O(CF₂ CF₂O)ₘ(CF₂O)ₙCF₂COF (MW=750; Mw/Mn 1.1 m/n=1.8, 67 mmol) and 35 g HFPO (MW=166, 211 mmol).

After removal of the low-boiling fluorinated by-products and solvents by distillation at room pressure, the distillation residue was submitted to thin layer distillation, to provide 67.8 g of a mixture containing (¹⁹F-NMR analysis):
- 96% (60.3 mmol) title compound;
- 3% of a compound containing -CF(CF₃)CF₂OCF(CF₃)COF end groups and
- 1% of a compound of formula: FC(O)CF₂O(CF₂CF₂O)ₘ(CF₂O)ₙCF₂CF₂ OCF(CF₃)COF.

The thin layer distillation residue (approx. 4.7 g) consisted of:
- 83% FC(O)CF(CF₃)OCF₂CF₂O(CF₂CF₂O)ₘ(CF₂O)ₙCF₂CF₂OCF(CF₃) CF₂OCF(CF₃)COF and
- 17% title compound.

The yield of title compound was 90.2% and the selectivity (defined as in Example 1, method 1) was 96%.

Tg (midpoint): -105°C

Conversion was therefore higher than 90%.

### Example 3 - Synthesis of CH₃CH₂OC(O)CF(CF₃)OCF₂CF₂O(CF₂CF₂O)ₘ (CF20)n CF₂CF₂O-CF(CF₃)COOCH₂CH₃ (MW 2060)

1.78 g CsF (MW= 152, 11.7 mmol), 70 g diethylene glycol dimethyl ether, 140 g bis-trifluoromethyl benzene and 48.4 g diacyl fluoride of formula FC(O)CF₂O(CF₂CF₂O)ₘ(CF₂O)ₙCF₂COF (MW=1650, Mw/Mn = 1.2, m/n=1.5, 29.3 mmol) were charged under nitrogen atmosphere in a 0.5 l AISI 316 reactor, equipped with agitator, sampling device, thermocouple and manometer. The resulting mixture was heated to 40°C and left under stirring for two hours, then cooled down to -25°C. Nitrogen was removed by connecting the reactor to a vacuum pump.

14 g HFPO (MW=166, 84.3 mmol) was added over 2 hours and the resulting mixture was left under stirring for 4 hours. After this time, the mixture was added with 30 g ethanol and 6 g triethylamine (59.4 mmol), keeping the temperature at -25°C; upon completion of the addition, the temperature was raised to 20°C and the resulting reaction solution was poured into a plastic separation funnel containing 50 ml water. Formation of an upper inorganic phase and a lower organic phase was observed; the latter was recovered and distilled at room pressure.

The following fractions were isolated:
- 3.0 g CF₃CF₋₂C(O)OCH₂CH₃ (b.p. 75-80°C);
- 130 g hexafluoroxylene (b.p.112°C);
- CF₃CF₂CF₋₂OCF(CF₃)C(O)OCH₂CH₃ (recovered at a temperature ranging from 140 to 150°C);
- 10 g diethylene glycol dimethyl ether (b.p.160°C).

The distillation residue was submitted to thin layer distillation at a temperature ranging from 200 to 270°C and at a pressure lower than 1.33 Pa, to provide 54.9 g (26.8 mmol) of title compound with 96% purity. The impurities consisted of:
- 2% wt CH₃CH₂OC(O)CF₂O(CF₂CF₂O)ₘ(CF₂O)ₙCF₂CF₂O-CF(CF₃ )C(O)OCH₂CH₃ (MW 1950) and
- 2% wt of a product having a terminal group containing one HFPO moiety of formula -OCF(CF₃)C(O)OCH₂CH₃ and a terminal group containing two HFPO moieties of formula -CF(CF₃)CF₂OCF(CF₃)C(O)OCH₂CH₃.

The thin layer distillation residue (approx. 5 g) was analysed by ¹⁹F-NMR. The results confirmed that this residue consisted of:
80% wt CH₃CH₂OC(O)CF(CF₃)OCF₂CF₂O(CF₂CF₂O)ₘ(CF₂O)ₙCF₂CF₂ OCF(CF₃) CF₂OCF(CF₃)C(O)OCH₂CH₃ (MW=2230) in admixture with 20% wt of title compound.

The yield of title compound with respect to the starting product amounted to 89%. The overall monoaddition selectivity (defined as in Example 1, method 1) was 96%.

Tg: - 108°C (midpoint).

Conversion was therefore higher than 90%.

### Example 4 - Synthesis of CH₃CH₂OC(O)CF(CF₃)OCF₂CF₂O(CF₂CF₂O)ₘ (CF₂O)ₙCF₂CF₂OCF(CF₃)C(O)OCH₂CH₃ (MW = 1112)

The title compound was prepared from the compound of example 2 following the procedure described in example 3 above.

### Example 5 - Synthesis of HOCH₂CF(CF₃)OCF₂CF₂O(CF₂CF₂O)ₘ(CF₂O)ₙCF₂CF₂OCF(CF₃)CH₂O H (MW 1970)

50.0 g of a 3% wt NaBH₄ (MW= 38, 39.5 mmol) solution in absolute ethanol was charged into a 4-neck round-bottom flask, equipped with agitator, thermometer, condenser and dropping funnel; thereafter, the compound of example 3 (40 g, 19.4 mmol) was added drop-by-drop, keeping the temperature between 10 and 15°C.

Upon completion of the addition, the mixture was left under stirring for 30 minutes, allowing the temperature to rise up to room temperature. After this time, 15 g of a 10% HCl solution was added to neutralise any NaBH₄ excess and to hydrolyse any boric esters. The resulting reaction crude was poured into a separation funnel to allow separation of two phases; the lower one was recovered and the solvent was removed at 80°C at a pressure of about 2.67 kPa (20 mmHg), to recover 36.3 g (18.4 mmol) title compound.

Yield: 94.9% with respect to the starting compound.

Tg: -105°C (midpoint).

Conversion was therefore higher than 90%.

### Example 6 - Synthesis of EtOC(O)CF(CF₃)O(CF₂CF₂O)₃CF(CF₃-)COOEt (MW= 710)

This compound was synthesised following the procedure described in example 3, using 5 g CsF (MW= 152, 33 mmol), 40 g diethylene glycol dimethyl ether, 80 g bis trifluoromethyl benzene, 25 g diacyl fluoride of formula FC(O)CF₂OCF₂CF₂OCF₂COF (MW 326, 77 mmol), 35 g HFPO (MW 166, 210 mmol) and 30 g ethanol. Upon completion of the synthesis, the resulting solution was poured into a plastic separation funnel containing 60 ml water. Separation of two phases was observed, an upper aqueous phase and a lower organic phase; the latter one was recovered and submitted to distillation, to provide 51 g of a mixture containing:
- 97% title compound and
- 3% of a compound containing one HFPO moiety at one end chain and a -CF(CF₃)CF₂OCF(CF₃)C(O)OCH₂CH₃ moiety at the other end chain.

The distillation residue (approx. 5 g) was analysed by ¹⁹F-NMR analysis and the results confirmed that it contained 85% of product containing one HFPO unit at one end chain and two moieties of formula -CF(CF₃)CF₂ OCF(CF₃)C(O)OCH₂CH₃ at the other end chain in admixture with 15% title compound.

The overall yield of title compound was approximately 90% and the selectivity of mono-addition (defined as in Example 1, method 1) was 95%.

### Example 7 - Synthesis of [(O)CCF(CF₃)OCF₂CF₂O (CF₂CF₂O)ₘ(CF₂O)ₙ CF₂CF₂OCF(CF₃)C(O)NH(CH₂)₆NH]z

The title compound, in which m and n are as defined in the description and z is an integer higher than 1, was prepared by reacting under vacuum the compound of example 3 with hexamethylenediamine in a 1:1 molar ratio at 150°C. The structure was confirmed by ¹⁹F-NMR and IR analysis.

### COMPARATIVE EXAMPLES

### Comparative example 1 - Synthesis of CH₃CH₂OC(O)CF(CF₃)OCF₂CF₂O(CF₂CF₂O)ₘ(CF₂O)ₙCF₂CF₂OCF(CF₃ ) C(O)OCH₂CH₃ (MW 2060) according to US 4053498 (US AIR FORCE)

1.85 g CsF (MW = 152, 12 mmol), 110 g diethylene glycol dimethyl ether, 50 g diacyl fluoride of formula: FC(O)CF₂O(CF₂CF₂O)ₘ(CF₂O)ₙCF₂COF (MW= 1650, Mw/Mn = 1.2, m/n=1.5, 30.3 mmol) were loaded in a 0.5 l 316 AISI reactor, equipped with agitator, sampling device, thermocouple and manometer under nitrogen atmosphere.

The resulting mixture was then heated to 40°C and left under stirring for two hours, then cooled to -25°C; nitrogen was removed by connecting the reactor to a vacuum pump. 15 g HFPO (MW=166, 90 mmol) was added over two hours and the resulting mixture was left under stirring for 4 hours. After this time, the temperature was allowed to rise to room temperature and the mixture was submitted to nitrogen purge cycles. Thereafter, the reaction crude was added with 40 g absolute ethanol and the reaction was continued according to example 3 above.

The low-boiling by-products and solvents were then removed by distillation at room temperature and the distillation residue was submitted to thin layer distillation under reduced pressure to afford 52.5 g of a mixture of products that, according to ¹⁹F-NMR analysis, had only 35% conversion and in which 30% of the end groups contained two or more HFPO moieties. As a consequence, the mixture contained only 5% of title compound, which could not be isolated in pure form.

### Comparative example 2 - Synthesis of CH₃CH₂OC(O)CF(CF₃)OCF₂CF₂ O(CF₂CF₂O)ₘ(CF₂O)ₙCF₂CF₂OCF(CF₃)C(O)OCH₂CH₃ (MW = 2060) according to US 4053498 (US AIR FORCE)

Synthesis and purification were carried out as described in comparative example 1 above, using 9.2 g (i.e. a higher amount with respect to that example) CsF (MW= 152, 61 mmol), 100 g diethylene glycol dimethyl ether, 50 g diacyl fluoride of formula FC(O)CF₂O(CF₂CF₂O)ₘ(CF₂O)ₙCF₂COF (MW= 1650, Mw/Mn = 1.2, m/n=1.5, 30.3 mmol) and 15 g HFPO (MW=166, 90 mmol).

Thin layer chromatography afforded 55 g of a mixture of products with 60% conversion, in which 30% of the reacted -COF groups of the diacyl fluoride precursor contained at least two HFPO moieties, as confirmed by ¹⁹F-NMR analysis. Statistically, 15% of this product consisted of the title compound which, however, could not be separated from the mixture.

### Comparative example 3 - Synthesis of CH₃CH₂OC(O)CF(CF₃)OCF₂CF₂ O(CF₂CF₂O)ₘ(CF₂O)ₙCF₂CF₂OCF(CF₃)C(O)OCH₂CH₃ (MW = 770) according to US 4053498 (US AIR FORCE) US 4,053,498

Synthesis and purification were carried out as described in comparative example 1, using 1 g CsF (MW = 152, 6.6 mmol), 100 g diethylene glycol dimethyl ether, 25 g of diacyl fluoride of formula FC(O)CF₂O(CF₂CF₂O)ₘ (CF₂O)ₙCF₂COF (MW = 750; Mw/Mn 1.1 m/n = 1.8, 33.3 mmol) and 32 g (193 mmol, i.e. a large excess) HFPO.

Thin layer chromatography afforded 50 g of a product which, from ¹⁹ F-NMR analysis, showed 99% conversion and wherein terminal groups statistically contained 2.5 HFPO units, i.e. a product of formula:

CH₃CH₂OC(O)CF(CF₃)[OCF₂CF(CF₃)]ₛOCF₂CF₂O(CF₂CF₂O)ₘ(CF₂O)ₙ CF₂CF₂O[CF(CF₃)CF₂O]CF(CF₃)C(O)OCH₂CH₃

wherein s = 2.5 and m and n were as defined above.

Accordingly, the product containing one HFPO moiety at each end of the polymer chain could not be separated from the mixture.

The Tg (midpoint of the mixture) was -78°C.

### Comparative example 4 - Synthesis of FC(O)CF(CF₃)OCF₂CF₂O(CF₂CF₂ O)ₘ(CF₂O)ₙCF₂CF₂OCF(CF₃)COF (MW 1960) according to US 2004/0116742 A1

1.20 g KF (MW = 58.1, 20.6 mmol) and 50 g diethylene glycol dimethyl ether were loaded, under nitrogen atmosphere, in a 0.5 l AISI 316 reactor equipped with agitator, thermocouple and sampling device and the resulting mixture was stirred at -17°C.

101 g diacyl fluoride of formula FC(O)CF₂O(CF₂CF₂O)ₘ(CF₂O)ₙCF₂COF (MW= 1650, Mw/Mn = 1.2, m/n=1.5, 61.2 mmol) was added and the resulting mixture was stirred for 30 minutes, then 15.2 g (92 mmol) HFPO was added to the reaction mass over a time period of one hour; a temperature increase up to -4°C was observed. After 30 minutes the internal temperature was raised to 20°C.

The reaction mass was unloaded from the reactor and the lower fluorinated phase (113 g) was separated and submitted to distillation. According to ¹⁹F-NMR analysis, this phase contained a mixture of products having only 25% unreacted end groups of formula -OCF₂COF; 69% of the product mixture consisted of the monoaddition product and 4% of the bis-addition product. This composition statistically corresponds to the following products:
a) 6% unreacted acyl fluoride precursor;
b) 50% title compound;
c) a mixture of FC(O)CF₂O(CF₂CF₂O)ₘ(CF₂O)ₙCF₂CF₂OCF(CF₃)COF (36%), FC(O)CF₂O(CF₂CF₂O)ₘ(CF₂O)ₙCF₂CF₂O[CF(CF₃)CF₂O]ₓCF(CF ₃)COF (2%), with x being equal to or higher than 1; FC(O)CF(CF₃)OCF₂CF₂O(CF₂CF₂O)ₘ(CF₂O)ₙCF₂CF₂O[CF(CF₃)CF₂O]ₓ CF(CF₃)COF (6%), with x being equal to or higher than 1.

The fluorinated phase was subjected to distillation; however, neither the title compound nor any other compounds could be isolated in pure form.

### Comparative example 5 - Synthesis of FC(O)CF(CF₃)OCF₂CF₂O(CF₂CF₂ O)ₘ(CF₂O)ₙ CF₂CF₂OCF(CF₃)COF (MW 1960) according to example 3 of US 2004/0116742 A1

The procedure described in comparative example 4 above was followed using a lower amount of HFPO; in fact, 8.6 g (52 mmol) HFPO was added instead of 15.2 g (92 mmol). The amounts of all other reagents and solvents remained unchanged.

The fluorinated phase (106 g) obtained upon completion of the reaction contained 60% unreacted acyl fluoride precursor with end groups of formula -OCF₂COF, 37% of mono-addition product with end groups of formula -CF(CF₃)COF and 3% of bis-addition product with end groups of formula -CF(CF₃)CF₂OCF(CF₃)COF. This composition statistically corresponds to the following mixture:
a) 36% unreacted acyl fluoride precursor;
b) 14% title compound;
c) a mixture of FC(O)CF₂O(CF₂CF₂O)ₘ(CF₂O)ₙCF₂CF₂OCF(CF₃)COF (44%), FC(O)CF₂O(CF₂CF₂O)ₘ(CF₂O)ₙCF₂CF₂O[CF(CF₃)CF₂O]ₓCF(CF ₃)COF (3.6%), with x being equal to or higher than 1; FC(O)CF(CF₃)OCF₂CF₂O(CF₂CF₂O)ₘ(CF₂O)ₙCF₂CF₂O[CF(CF₃)CF₂O]ₓ CF(CF₃)COF (2.2 %), with x being equal to or higher than 1.

The fluorinated phase was subjected to distillation; however, neither the title compound nor any other compounds could be isolated in pure form.

### Comparative example 6 - Synthesis of CF₃CF₂O(CF₂CF₂O)ₘ(CF₂O)ₙCF₂ CF₂OCF(CF₃)COF (MW 1166) according to example 3 of US 2004/0116742 A1

The procedure described in comparative example 4 above was followed using the following amounts of reagents and solvents:
2.0 g KF (MW = 58.1 34.4 mmol), 50 g diethylene glycol dimethyl ether, 100 g diacyl fluoride of formula FC(O)CF₂O(CF₂CF₂O)m(CF₂O)ₙCF₂COF (MW = 1000, Mw/Mn = 1.3, m/n = 1.5, 100 mmol) and 12.3 g (74 mmol) HFPO.

Upon completion of the reaction, the lower fluorinated phase obtained (107 g) contained a mixture of products having 30% unreacted -OCF₂COF end groups, 65% end groups of formula -CF(CF₃)COF and 4% groups of formula -CF(CF₃)CF₂OCF(CF₃)COF.

This phase was subjected to distillation; however, it was not possible to isolate the desired title compound, due to the fact that polydispersivity is high and does not allow to differentiate the vapour tension of the phase components.

### Comparative example 7 - Synthesis of CF₃O(CF₂CF₂O)ₘ(CF₂O)ₙCF₂CF₂ OCF(CF₃)COF (MW 966) according to example 3 of US 2004/0116742 A1

The procedure described in comparative example 4 above was followed using the following amounts of reagents and solvents:
1.20 g KF (MW = 58.1, 20.6 mmol), 50 g diethylene glycol dimethyl ether, 96 g acyl fluoride of formula CF₃O(CF₂CF₂O)ₘ(CF₂O)ₙCF₂COF (MW = 800, Mw/Mn = 1.4, m/n=1.5, 120 mmol) and 15.2 g (92 mmol) HFPO.

The lower fluorinated fraction (105 g), obtained upon completion of the reaction contained a mixture of products having 30% unreacted -OCF₂ COF end groups, 60% of monoaddition product and 8% bis-addition product with end groups of formula -CF(CF₃)CF₂OCF(CF₃)COF.

This fraction was submitted to distillation, but the desired title compound could not be isolated.

### HYDROLYSIS TESTS

### Test 1 - Resistance to hydrolysis of the ester of example 4 at neutral pH

Resistance to hydrolysis of this ester was evaluated at neutral pH as described in general procedure 1.3 above in comparison with CH₃CH₂ OC(O)CF₂O(CF₂CF₂O)ₘ(CF₂O)ₘCF₂C(O)OCH₂CH₃ (reference compound), obtained from FC(O)CF₂O(CF₂CF₂O)ₘ(CF₂O)ₙCF₂COF (MW = 750).

The title diethyl ester underwent < 0.1 % hydrolysis, while about 45% reference compound was hydrolysed.

### Test 2 - Resistance to hydrolysis of the ester of example 4 at basic pH

Resistance to hydrolysis of this ester was evaluated at basic pH as described above in comparison with CH₃CH₂OC(O)CF₂O(CF₂CF₂O)ₘ(CF ₂O)ₙCF₂C(O)OCH₂CH₃ (reference compound), obtained from FC(O)CF₂ O(CF₂CF₂O)ₘ(CF₂0)ₙCF₂COF (MW = 750).

The title diethyl ester underwent < 5% hydrolysis, while about 40% reference compound was hydrolysed.

### Test 3 - Resistance to hydrolysis of the polyamide of example 7

Resistance to hydrolysis of the polyamide of example 7 (in the present example referred to as polyamide A) was determined in comparison with a reference polyamide of formula: [(O)CCF₂O(CF₂CF₂O)ₘ(CF₂O)ₙCF₂ C(O)NH(CH₂)₆NH-]_{z}- (polyamide B), which was obtained by polycondesation reaction of the diethyl ester of FC(O)CF₂O(CF₂CF₂O)ₘ (CF₂O)ₙCF₂COF (MW= 1650, Mw/Mn = 1.1, m/n=1.5) with an equimolar amount of hexamethylenediamine, according to the procedure reported in example 7.

### Polyamides A and B were submitted to hydrolysis at basic pH for 3 weeks, then the hydrolysis percentage was evaluated through acid/base titration. Polyamide A underwent < 1% hydrolysis, while polyamide B underwent about 8% hydrolysis. This demonstrated that the polyamide according to the present invention is eight times more stable than a polyamides obtained from a diacyl fluoride precursor that does not contain HFPO terminal units.

### Test 4 - Resistance to hydrolysis of the diacetate of the compound of example 5 at basic pH

The alcohol compound of example 5 was transformed into the corresponding diacetate by reaction with acetic anhydride according to a known procedure. This diacetate was subjected to hydrolysis at basic pH as described in general procedure 1.3 above. After 7 hours, less than 1% hydrolysis was observed.

Similarly, Fluorolink^{®} D PFPE from Solvay Solexis was transformed into the corresponding diacetate (chain ends of formula -CF₂CH₂OC(O)CH₃) and submitted to hydrolysis under the same conditions. After 7 hours, 5% hydrolysis was observed.

### Test 5 - Resistance to hydrolysis of the diethyl ester of example 6 at neutral pH

The diethyl ester of example 6 was submitted to a comparative test of resistance to hydrolysis with respect to the diethyl ester of formula CH₃CH ₂OC(O)CF₂O(CF₂CF₂O)₃CF₂C(O)OCH₂CH₃ (reference compound), prepared from a diacyl precursor of formula: FC(O)CF₂O(CF₂CF₂O)₃CF₂ COF.

After about 7 hours at 70°C less than 5% diethyl ester of example 6 underwent hydrolysis, while more than 95% reference compound underwent hydrolysis.

## Claims

1. Compounds of formula (I)
X-O-R_{f}-CF₂CF₂O-[CF(CF₃)CF₂O]ₓ-CF(CF₃)-COF (I)
wherein:
- R_{f} is a linear (per)fluoropolyoxyalkylene chain comprising repeating units R°, randomly distributed along the (per)fluoropolyoxyalkylene chain, selected from the group consisting of:
(i) -CF₂O-;
(ii) -CF₂CF₂O-;
(iii) -CF₂CF₂CF₂O-;
(iv) -CF₂CF₂CF₂CF₂O-;
- X is -CF₂CF₂O-[CF(CF₃)CF₂O]ₓ-CF(CF₃)-COF or a (per)fluoroalkyl chain containing from 1 to 3 carbon atoms, optionally containing 1 chlorine, bromine or hydrogen atom;
- x is 0 or an integer equal to or higher than 1.

2. Compounds according to claim 1 in which R_{f} is a linear (per)fluoropolyether chain complying with formula (CF₂O)ₙ(CF₂CF₂O)ₘ, in which m and n are 0 or integers equal to or higher than 1, with the proviso that at least one of m and n is other than 0.

3. Compounds of formula (III):
X²-O-R_{f}-CF₂CF₂O-[CF(CF₃)CF₂O]ₓ-CF(CF₃)-(A)_{q}-(T)ₚ (III)
in which:
- X² is -CF₂CF₂O-[CF(CF₃)CF₂O]ₓ-CF(CF₃)-(A)_{q}-(T)ₚ or a perfluoroalkyl chain containing from 1 to 3 carbon atoms, optionally containing 1 chlorine, bromine or hydrogen atom;
- R_{f} and x are as defined in claim 1 or 2;
- q and p are 0 or 1, with the proviso that at least one of them is other than 0;
- A is a straight or branched alkyl chain, an (alkyl)clycloaliphatic or an (alkyl)aromatic chain when p is 0 or a straight or branched alkylene chain, an (alkylene)clycloaliphatic or an (alkylene)aromatic chain when p is 1;
- T is a group consisting of or containing one or more groups selected from hydroxyl, ester, carboxy, thio, alkylthio, amino, alkylamino, a silicon-containing group, cyano, isocyanate, vinyl, vinyl ether, vinylalkyl ether, aldehyde, keto, sulphonyl, sulphate, phosphonate, phosphate, carbonate, anhydride, halogen, oxime, hydrazine, guanidine, amido, acrylate, methacrylate, nitro and epoxide.

4. Compounds according to claim 3 in which A is selected from: a straight or branched alkyl or alkylene chain containing from 1 to 20 carbon atoms; an (alkyl)cycloaliphatic or (alkylene)cycloaliphatic chain in which the alkyl or alkylene moiety is a straight or branched alkyl or alkylene moiety containing from 1 to 20 carbon atoms and the cycloaliphatic moiety has from 3 to 20 carbon atoms; an (alkyl)aromatic or (alkyene)aromatic chain in which the alkyl or alkylene moiety is as defined above and the aromatic moiety contains 5 to 20 carbon atoms, wherein the alkyl or alkylene chain optionally contains one or more -COO-, -COS- or -CONH- groups and/or one or more etheroatoms selected from N, P, S and O and in the cycloaliphatic or aromatic moiety one ore more carbon atoms is optionally replaced by etheroatoms selected from N, P,SandO.

5. Compounds according to claim 3 or 4 in which T is selected from:
- OH;
- CH(CH₂OH)₂;
- COOR' in which R' is a straight or branched alkyl chain containing from 1 to 20 carbon atoms and optionally containing fluorine atoms and/or a 3 to 20 member cycloaliphatic or heterocycloaliphatic moiety or a 5 to 20 member aromatic or heteroaromatic moiety;
- CH(COOR')₂, in which R' is as defined above;
- COOH, -CH(COOH)₂ and their respective alkali, alkaline-metal salts or acyl halides;
- SH;
- SR', in which R' is as defined above;
- SiR'*a*Q_{3-d} in which R' is as defined above, *d* is 0 or an integer from 1 to 3, Q is an -O(CO)*d0*R' group and *d0* is 0 or 1;
- CN;
- CH(CN)₂;
- O(CH₂)_{m'}CH=CH₂, in which m' ranges from 1 to 18;
- CH[O(CH₂)_{m'}CH=CH₂]₂, in which m' is as defined above, more preferably a group of formula -CH[OCH₂CH=CH₂]₂;
- NH2;
- NHR' or -NR'₂, in which R' is as defined above;
- CH(CH₂NHR')₂ or -CH(CH₂NR'₂)₂, in which R' is as defined above;
- CHO;
- COR', in which R' is as defined above;
- SO₃H and any alkali or alkaline-hearth metal salts thereof;
- SO₂Hal, in which Hal is halogen selected from fluorine, chlorine, bromine and iodine;
- SO₂R', in which R' is as defined above;
- SO₂R", in which R" is a fluorinated straight or branched C₁-C₆alkyl chain;
- OSO₃H and any alkali or alkaline-hearth metal salts thereof,
- OSO₂R', in which R' are as defined above;
- P(O)(OH)₂ and -P(O)(OH)(OR') and their respective alkali or alkaline-hearth metal salts;
- P(O)(OR')₂, in which R' is as defined above;
- OP(O)(OH)₂; -OP(O)(OH)(OR') in which R' is as defined above and their respective alkali or alkali-earth metal salts;
- OP(O)(OR')₂, in which R' is as defined above.
- OC(O)OH and its alkali or alkali-earth metal salts, -OC(O)Hal in which Hal is as defined above and -OC(O)OR' in which R' is as defined above;
- halogen selected from fluorine, chlorine, bromine and iodine;
- (O)C-O-C(O)-R', in which R' is as defined above;
- NH-OH or -NH-OR', in which R' is as defined above;
- NH-NH₂ or -NH-NHR', in which R' is as defined above;
- NH-C(=NH)-NH₂ or a group of formula -NH-C(=NH)-NHR', in which R' is as defined above.

6. Compounds according to claim 5 selected from: compounds of formula (IIIa):
X²-O-R_{f}-CF₂CF₂O-[CF(CF₃)CF₂O]ₓ-CF(CF₃)-COOR' (IIIa)
and compounds of formula (IIId):
X²-O-R_{f}-CF₂CF₂O-[CF(CF₃)CF₂O]ₓ-CF(CF₃)-CH₂OH (IIId)
in which:
- X², R_{f} and x are as defined in claim 1 or 2 and
- R' is as defined in claim 5.

7. Manufactured or synthetic articles treated with a compound of any one of claims 3 to 6.

8. A process for the preparation of compounds of formula (I) as defined in claim 1 or 2 which comprises reacting a compound of formula (II):
X¹-O-R_{f}-CF₂COF (II)
in which:
- R_{f} is as defined in claim 1 or 2 and
- X¹ is CF₂COF or a (per)fluoroalkyl chain containing from 1 to 3 carbon atoms, optionally containing 1 chlorine, bromine or a hydrogen atom with hexafluoropropylene oxide in the presence of an inorganic or organic fluoride as catalyst and of a mixture of a fluorinated solvent and an oxygen-containing hydrogenated solvent.

9. A process according to claim 8 wherein the weight ratio of fluorinated solvent to oxygen-containing hydrogenated solvent ranges from 0.1 to 10.

10. A process according to claim 8 or 9 in which the equivalent ratio between the compound of formula (II) and hexafluoropropylene oxide ranges from 1:1.1 to 1: 3.1.

## Patentansprüche

1. Verbindungen der Formel (I)
X-O-R₁-CF₂CF₂O-[CF(CF₂)CF₂O]ₓ-CF(CF₃)-COF (I)
wobei:
- R_{f} eine lineare (Per)fluorpolyoxyalkylenkette ist, die sich wiederholende Einheiten R° umfasst, die statistisch entlang der (Per)fluopolyoxyalkylenkette verteilt sind, ausgewählt aus der Gruppe bestehend aus :
(i) -CF₂O-;
(ii) -CF₂CF₂O-;
(iii) -CF₂CF₂CF₂O-;
(iv) -CF₂CF₂CF₂CF₂O-;
- X -CF₂CF₂O-[CF (CF₃)CF₂O]ₓ-CF (CF₃)-COF oder eine (Per)fluoralkylkette ist, die 1 bis 3 Kohlenstoffatome enthält, die gegebenenfalls 1 Chlor- Brom- oder Wasserstoffatom enthalten;
- x 0 oder eine ganze Zahl gleich oder größer als 1 ist.

2. Verbindungen nach Anspruch 1, wobei R_{f} eine lineare (Per)fluorpolyetherkette ist, die der Formel (CF₂O)ₙ(CF₂CF₂O)ₘ entspricht, wobei m und n 0 oder ganze Zahlen gleich oder größer als 1 sind, mit der Maßgabe, dass mindestens einer von m und n von 0 verschieden ist.

3. Verbindungen der Formel (III) :
X²-O-R_{f}-CF₂CF₂O-[CF(CF₃)CF₂O]ₓ-CF(CF₃)-(A)_{q}-(T)ₚ (III)
wobei:
- X² -CF₂CF₂O-[CF(CF₃)CF₂O]ₓ-CF(CF₃)-(A)_{q}-(T)ₚ oder eine Perfluoralkylkette ist, die 1 bis 3 Kohlenstoffatome enthält, die gegebenenfalls 1 Chlor-, Brom- oder Wasserstoffatom enthalten;
- R_{f} und x wie in Anspruch 1 oder 2 definiert sind;
- q und p 0 oder 1 sind, mit der Maßgabe, dass mindestens einer von ihnen von 0 verschieden ist;
- A eine geradkettige oder verzweigte Alkylkette, eine (alkyl)cycloaliphatische oder eine (alkyl)aromatische Kette ist, wenn p 0 ist, oder eine geradkettige oder verzweigte Alkylenkette, eine (alkylen)cycloaliphatische oder eine (alkylen)aromatische Kette ist, wenn p 1 ist;
- T eine Gruppe ist, die aus einer oder mehreren Gruppen ausgewählt aus Hydroxyl, Ester, Carboxy, Thio, Alkylthio, Amino, Alkylamino, einer silicium-haltigen Gruppe, Cyano, Isocyanat, Vinyl, Vinylether, Vinylalkylether, Aldehyd, Keto, Sulfonyl, Sulfat, Phosphonat, Phosphat, Carbonat, Anhydrid, Halogen, Oxim, Hydrazin, Guanidin, Amido, Acrylat, Methacrylat, Nitro und Epoxid besteht oder diese enthält.

4. Verbindungen nach Anspruch 3, wobei A ausgewählt ist aus einer geradkettigen oder verzweigten Alkyl- oder Alkylenkette, die 1 bis 20 Kohlenstoffatome enthält; einer (alkyl)cycloaliphatischen oder (alkylen)cycloaliphatischen Kette, in der der Alkyl- oder Alkylenanteil ein geradkettiger oder verzweigter Alkyl- oder Alkylenanteil ist, der 1 bis 20 Kohlenstoffatome enthält und der cycloaliphatische Anteil 3 bis 20 Kohlenstoffatome aufweist; einer (alkyl)aromatischen oder (alkyen)aromatischen Kette, wobei der Alkyl- oder Alkylenanteil wie oben definiert ist und der aromatische Anteil 5 bis 20 Kohlenstoffatome enthält, wobei die Alkyl- oder Alkylenkette gegebenenfalls ein oder mehrere -COO-, -COS- oder -CONH-Gruppen und/oder ein oder mehrere Heteroatome ausgewählt aus N, P, S und O enthält und in dem cycloaliphatischen oder aromatischen Anteil ein oder mehrere Kohlenstoffatome gegebenenfalls durch Heteroatome ausgewählt aus N, P, S und O ersetzt sind.

5. Verbindungen nach Anspruch 3 oder 4, wobei T ausgewählt ist aus :
- OH;
- CH(CH₂OH)₂;
- COOR', wobei R' eine geradkettige oder verzweigte Alkylkette ist, die 1 bis 20 Kohlenstoffatome enthält und gegebenenfalls Fluoratome und/oder einen 3- bis 20-gliedrigen cycloaliphatischen oder heterocycloaliphatischen Anteil oder einen 5- bis 20-gliedrigen aromatischen oder heteroaromatischen Anteil enthält;
- CH(COOR')₂, wobei R' wie oben definiert ist;
- COOH, -CH(COOH)₂ und ihre jeweiligen Alkali-, Erdalkalimetallsalze oder Acylhalogenide;
- SH;
- SR', wobei R' wie oben definiert ist;
- SiR'*_{d}*Q_{3-d}, wobei R' wie oben definiert ist, *d* 0 oder eine ganze Zahl von 1 bis 3 ist, Q eine -O(CO)*_{d0}*R' Gruppe ist und *d0* 0 oder 1 ist;
- CN;
- CH(CN)₂;
- O(CH₂)_{m'}-CH=CH₂, wobei m' im Bereich von 1 bis 18 liegt;
- CH[O(CH₂)ₘCH=CH₂]₂, wobei m' wie oben definiert ist, insbesondere eine Gruppe mit der Formel -CH[OCH₂CH=CH₂]₂;
- NH₂;
- NHR' oder -NR'₂, wobei R' wie oben definiert ist;
- CH(CH₂NHR')₂ oder -CH(CH₂NR'₂)₂, wobei R' wie oben definiert ist;
- CHO;
- COR', wobei R' wie oben definiert ist;
- SO₃H und jeglichen Alkali- oder Erdalkalimetallsalzen davon;
- SO₂Hal, wobei Hal Halogen ausgewählt aus Fluor, Chlor, Brom und Iod ist;
- SO₂R', wobei R' wie oben definiert ist;
- SO₂R", wobei R" eine fluorierte geradkettige oder verzweigte C₁-C₆-Alkylkette ist;
- OSO₃H und jeglichen Alkali- oder Erdalkalimetallsalzen davon;
- OSO₂R', wobei R' wie oben definiert ist;
- P(O)(OH)₂ und -P(O)(OH)(OR') und ihren jeweiligen Alkali- oder Erdalkalimetallsalzen;
- P(O)(OR')₂, wobei R' wie oben definiert ist;
- OP(O)(OH)₂; -OP(O)(OH)(OR'), wobei R' wie oben definiert ist, und ihren jeweiligen Alkali- oder Erdalkalimetallsalzen;
- OP(O)(OR')₂, wobei R' wie oben definiert ist;
- OC(O)OH und deren Alkali- oder Erdalkalimetallsalze, -OC(O)Hal, wobei Hal wie oben definiert ist, und -OC(O)OR', wobei R' wie oben definiert ist;
- Halogen ausgewählt aus Fluor, Chlor, Brom und Iod;
- (O)C-O-C(O)-R', wobei R' wie oben definiert ist;
- NH-OH oder -NH-OR', wobei R' wie oben definiert ist;
- NH-NH₂ oder -NH-NHR', wobei R' wie oben definiert ist;
- NH-C(=NH)-NH₂ oder einer Gruppe mit der Formel -NH-C(=NH)-NHR', wobei R' wie oben definiert ist.

6. Verbindungen nach Anspruch 5 ausgewählt aus :
Verbindungen der Formel (IIIa) :
X²-O-R_{f}-CF₂CF₂O- [CF (CF₅) CF₂O]ₓ-CF(CF₃)-COOR' (IIIa)
und Verbindungen der Formel (IIId):
X²-O-R_{f}-CF₂CF₂O-[CF(CF₃)CF₂O]ₓ-CF(CF₃)-CH₂OH (IIId)
wobei:
- X^{2,} R_{f} und x wie in Anspruch 1 oder 2 definiert sind, und
- R' wie in Anspruch 5 definiert ist.

7. Gefertigte oder synthetische Artikel, die mit einer Verbindung nach einem der Ansprüche 3 bis 6 behandelt sind.

8. Verfahren zur Herstellung von Verbindungen der Formel (I) wie in Anspruch 1 oder 2 definiert, umfassend Umsetzen einer Verbindung der Formel (II) :
X¹-O-R_{f}-CF₂COF (II)
wobei:
- R_{f} wie in Anspruch 1 oder 2 definiert ist, und
- X¹ CF₂COF oder eine (Per)fluoralkylkette ist, die 1 bis 3 Kohlenstoffatome enthält, die gegebenenfalls 1 Chlor-, Brom- oder Wasserstoffatom enthalten,
mit Hexafluorpropylenoxid in Gegenwart eines anorganischen oder organischen Fluorids als Katalysator und einer Mischung eines fluorierte Lösungsmittels und eines sauerstoffhaltigen hydrierten Lösungsmittel.

9. Verfahren nach Anspruch 8, wobei das Gewichtsverhältnis von fluoriertem Lösungsmittel zu sauerstoffhaltigem hydriertem Lösungsmittel im Bereich von 0,1 bis 10 liegt.

10. Verfahren nach Anspruch 8 oder 9, wobei das Äquivalenzverhältnis zwischen der Verbindung der Formel (II) und Hexafluorpropylenoxid im Bereich von 1:1,1 bis 1:3,1 liegt.

## Revendications

1. Composés de formule (I)
X-O-R_{f}-CF₂CF₂O-[CF(CF₃)CF₂O]ₓ-CF(CF₃)-COF (I)
dans laquelle :
- R_{f} est une chaîne (per)fluoropolyoxyalkylène linéaire comprenant des motifs répétitifs R°, distribués au hasard le long de la chaîne (per)fluoropolyoxyalkylène, choisis dans le groupe consistant en :
(i) -CF₂O- ;
(ii) -CF₂CF₂O-;
(iii) -CF₂CF₂CF₂O- ;
(iv) -CF₂CF₂CF₂CF₂O- ;
- X est -CF₂CF₂O-[CF(CF₃)CF₂O]ₓ-CF(CF₃)-COF, ou une chaîne (per)fluoralkyle contenant de 1 à 3 atomes de carbone, en option contenant 1 atome de chlore, de brome ou d'hydrogène ;
- x vaut 0 ou est un entier supérieur ou égal à 1.

2. Composés selon la revendication 1, dans lequel R_{f} est une chaîne (per)fluoropolyéther linéaire correspondant à la formule (CF₂O)ₙ(CF₂CF₂O)ₘ, dans laquelle m et n valent 0 ou sont des entiers supérieurs ou égaux à 1, à la condition qu'au moins l'un de m et de n soit différent de 0.

3. Composés de formule (III) :
X²-O-R_{f}-CF₂CF₂O-[CF(CF₃)CF₂O]ₓ-CF(CF₃)-(A)_{q}-(T)ₚ (III)
dans laquelle :
- X² est -CF₂CF₂O-[CF(CF₃)CF₂O]ₓ-CF(CF₃)-(A)_{q}-(T)ₚ ou une chaîne perfluoralkyle contenant de 1 à 3 atomes de carbone, en option contenant 1 atome de chlore, de brome, ou d'hydrogène ;
- R_{f} et x sont tels que définis dans la revendication 1 ou 2 ;
- q et p valent 0 ou 1, à la condition qu'au moins l'un d'entre eux soit différent de 0;
- A est une chaîne alkyle droite ou ramifiée, une chaîne (alkyl)cycloaliphatique ou une chaîne (alkyl)aromatique quand p vaut 0, ou une chaîne alkylène droite ou ramifiée, une chaîne (alkylène)cycloaliphatique ou une chaîne (alkylène)aromatique quand p vaut 1 ;
- T est un groupe consistant en un ou plusieurs groupes choisis parmi les groupes hydroxyle, ester, carboxy, thio, alkylthio, amino, alkylamino, un groupe contenant du silicium, cyano, isocyanate, vinyle, vinyléther, vinylalkyléther, aldéhyde, céto, sulfonyle, sulfate, phosphonate, phosphate, carbonate, anhydride, halogéno, oxime, hydrazine, guanidine, amido, acrylate, méthacrylate, nitro et époxyde, ou les contenant.

4. Composés selon la revendication 3, dans lesquels A est choisi parmi une chaîne alkyle ou alkylène droite ou ramifiée contenant de 1 à 20 atomes de carbone ; une chaîne (alkyl)cycloaliphatique ou (alkylène)cycloaliphatique dans laquelle le fragment alkyle ou alkylène est un fragment alkyle ou alkylène à chaîne droite ou ramifiée contenant de 1 à 20 atomes de carbone et le fragment cycloaliphatique a de 3 à 20 atomes de carbone ; une chaîne (alkyl)aromatique ou (alkylène)aromatique dans laquelle le fragment alkyle ou alkylène est tel que défini ci-dessus, et le fragment aromatique contient 5 à 20 atomes de carbone, la chaîne alkyle ou alkylène contenant en option un ou plusieurs groupes -COO-, -COS- ou -CONH- et/ou un ou plusieurs hétéroatomes choisis parmi N, P, S et O, et, dans le fragment cycloaliphatique ou aromatique, un ou plusieurs atomes de carbone étant en option remplacés par des hétéroatomes choisis parmi N, P, S ou O.

5. Composés selon la revendication 3 ou 4, dans lesquels T est choisi parmi :
- OH ;
- CH(CH₂OH)₂ ;
- COOR' où R' est une chaîne alkyle droite ou ramifiée contenant de 1 à 20 atomes de carbone et en option contenant des atomes de fluor, et/ou un fragment cycloaliphatique ou hétérocycloaliphatique à 3 à 20 chaînons, ou un fragment aromatique ou hétéroaromatique à 5 à 20 chaînons ;
- CH(COOR')₂, où R' est tel que défini ci-dessus ;
- COOH, -CH(COOH)₂ et leurs sels respectifs de métaux alcalins, de métaux alcalino-terreux, ou leurs halogénures d'acyle ;
- SH ;
- SR', où R' est tel que défini ci-dessus ;
- SiR'*_{d}*Q_{3-d}, où R' est tel que défini ci-dessus, *d* vaut 0 ou est un entier de 1 à 3, Q est un groupe -O(CO)*_{d0}*R', et *d0* vaut 0 ou 1 ;
- CN ;
- CH(CN)₂ ;
- O(CH₂))ₘCH=CH₂, où m'est compris dans la plage de 1 à 18 ;
- CH[O(CH₂)ₘCH=CH₂]₂ où m'est tel que défini ci-dessus, plus particulièrement un groupe de formule -CH[OCH₂CH=CH₂]₂ :
- NH₂;
- NHR' ou -NR'₂, où R' est tel que défini ci-dessus ;
- CH(CH₂NHR')₂ ou -CH(CH₂NR'₂)₂, où R' est tel que défini ci-dessus ;
- CHO ;
- COR', où R' est tel défini ci-dessus ;
- SO₃H, et tous sels de métaux alcalins ou alcalino-terreux de ce dernier ;
- SO₂Hal, où Hal est un halogène choisi parmi le fluor, le chlore, le brome et l'iode ;
- SO₂R', où R' est tel que défini ci-dessus ;
- SO₂R", où R" est une chaîne alkyle en C₁-C₆ droite ou ramifiée, fluorée ;
- OSO₃H et tous sels de métaux alcalins ou alcalino-terreux de ce dernier,
- OSO₂R', où R' est tel que défini ci-dessus ;
- P(O)(OH)₂ et -P(O)(OH)(OR') et leurs sels respectifs de métaux alcalins ou alcalino-terreux ;
- P(O)(OR')₂, où R' est tel que défini ci-dessus ;
- OP(O)(OH)₂ ; -OP(O)(OH)(OR'), où R' est tel que défini ci-dessus, et leur sels respectifs de métaux alcalins ou alcalino-temeux ;
- OP(O)(OR')₂, où R' est tel que défini ci-dessus ;
- OC(O)OH et ses sels de métaux alcalins ou alcalino-terreux, -OC(O)Hal où Hal est tel que défini ci-dessus et -OC(O)OR' où R' est tel que défini ci-dessus ;
- halogéno, choisi parmi les groupes fluoro, chloro, bromo et iodo ;
- (O)C-O-C(O)-R', où R' est tel que défini ci-dessus ;
- NH-OH ou -NH-OR', où R' est tel que défini ci-dessus ;
- NH-NH₂ ou -NH-NHR', où R' est tel que défini ci-dessus ;
- NH-C(=NH)-NH₂ ou un groupe de formule -NH-C(=NH)-NHR', où R' est tel que défini ci-dessus.

6. Composés selon la revendication 5, choisis parmi :
les composés de formule (IIIa) :
X²-O-R_{f}-CF₂CF₂O-[CF(CF₃)CF₂O]ₓ-CF(CF₃)-COOR' (IIIa)
et les composés de formule (IIId) :
X²-O-R_{f}-CF₂CF₂O-[CF(CF₃)CF₂O]ₓ-CF(CF₃)-CH₂OH (IIId)
dans lesquelles :
- X², R_{f} et x sont tels que définis dans la revendication 1 ou 2, et
- R' est tel que défini dans la revendication 5.

7. Articles fabriqués ou synthétiques traités avec un composé selon l'une quelconque des revendications 3 à 6.

8. Procédé de préparation de composés de formule (I) tels que définis dans la revendication 1 ou 2, qui comprend la réaction d'un composé de formule (II) :
X¹-O-R_{f}-CF₂COF (II)
dans laquelle :
- R_{f} est tel que défini dans la revendication 1 ou 2, et
- X¹ est CF₂COF ou une chaîne (per)fluoralkyle contenant de 1 à 3 atomes de carbone, en option contenant un atome de chlore, de brome ou d'hydrogène,
avec de l'oxyde d'hexafluoropropylène en présence d'un fluorure inorganique ou organique servant de catalyseur et d'un mélange d'un solvant fluoré et d'un solvant hydrogéné contenant de l'oxygène.

9. Procédé selon la revendication 8, dans lequel le rapport en poids du solvant fluoré au solvant hydrogéné contenant de l'oxygène est compris dans la plage de 0,1 à 10.

10. Procédé selon la revendication 8 ou 9, dans lequel le rapport entre équivalents, entre le composé de formule (II) et l'oxyde d'hexafluoropropylène, est compris dans la plage de 1:1,1 à 1:3,1.
